# EUROPÄISCHE PATENTSCHRIFT

(11) **EP 0 778 824 B1**
(45) Veröffentlichungstag und Bekanntmachung des Hinweises auf die Patenterteilung: **04.11.1998**
(21) Anmeldenummer: 95931165.5
(22) Anmeldetag: 19.08.1995
(51) Int. Cl.: C07C 271/22, A01N 47/10

(54) **CARBAMOYLCARBONSÄUREAMIDE**
CARBAMOYL CARBOXYLIC ACID AMIDES
AMIDES D'ACIDE CARBAMOYL-CARBOXYLIQUE

(30) Priorität: 03.09.1994 DE 4431467
(43) Veröffentlichungstag der Anmeldung: 18.06.1997
(73) Patentinhaber: BASF AKTIENGESELLSCHAFT, 67056 Ludwigshafen (DE)
(72) Erfinder: WETTERICH, Frank, D-64297 Darmstadt (DE); WAGNER, Oliver, D-66450 Bexbach (DE); EICKEN, Karl, D-67157 Wachenheim (DE); AMMERMANN, Eberhard, D-64646 Heppenheim (DE); LORENZ, Gisela, D-67434 Hambach (DE)
(86) Internationale Anmeldenummer: EP9503303
(87) Internationale Veröffentlichungsnummer: WO9607638

(56) Entgegenhaltungen:
- EP-A- 0 398 072
- DE-A- 4 321 897

## Beschreibung

Die vorliegende Erfindung betrifft Carbamoylcarbonsäureamide der allgemeinen Formel I 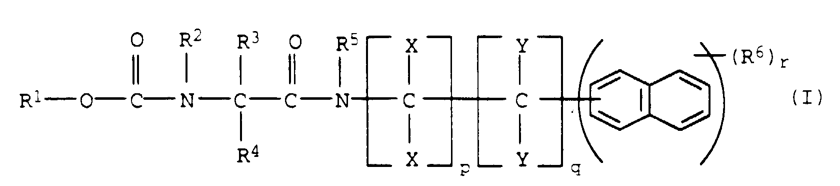 sowie deren Salze, in denen die Variablen die folgende Bedeutung haben:
- R¹: C₁-C₈-Alkyl, C₂-C₈-Alkenyl oder C₂-C₈-Alkinyl, wobei diese Reste partiell oder vollständig halogeniert sein und/oder eine bis drei der folgenden Gruppen tragen können: Cyano, C₁-C₄-Alkoxy, C₁-C₄-Halogenalkoxy, C₁-C₄-Alkylthio, C₁-C₄-Alkoxycarbonyl, C₃-C₇-Cycloalkyl, C₃-C₇-Cycloalkenyl, Aryl, Aryloxy und Heteroaryl, wobei die cyclischen und aromatischen Ringe dieser Gruppen einen bis drei der folgenden Substituenten tragen können: Halogen, Cyano, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Alkoxycarbonyl, Aryl, Aryloxy und Heteroaryl,
C₃-C₇-Cycloalkyl oder C₃-C₇-Cycloalkenyl, wobei diese Reste partiell oder vollständig halogeniert sein und/oder eine bis drei der folgenden Gruppen tragen können: Cyano, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Alkoxycarbonyl, Aryl, Aryloxy und Aryl-(C₁-C₄)-alkyl, wobei die aromatischen Ringe dieser Gruppen ihrerseits einen bis drei der folgenden Substituenten tragen können: Halogen, Cyano, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Alkoxycarbonyl, Aryl und Aryloxy,
einen nicht-aromatischen 4- bis 8-gliedrigen Ring, welcher als Ringglieder neben Kohlenstoff noch eines oder zwei der Heteroatome Sauerstoff, Schwefel und Stickstoff enthalten kann, wobei die Kohlenstoffatome im Ring eine oder zwei der folgenden Gruppen tragen können: Halogen, Cyano, C₁-C₄-Alkyl, C₁-C₄-Alkoxyalkyl, C₁-C₄-Halogenalkyl, C₁-C₄-Alkoxy,
C₁-C₄-Halogenalkoxy, C₁-C₄-Alkylthio, C₁-C₄-Alkoxycarbonyl, Aryl und Aryloxy und wobei das zweite und jedes weitere Stickstoffatom als Heteroatom im Ring Wasserstoff oder eine C₁-C₄-Alkylgruppe trägt;
- R²: Wasserstoff, C₁-C₈-Alkyl oder C₃-C₇-Cycloalkyl, welche partiell oder vollständig halogeniert sein können:
- R³: C₁-C₈-Alkyl, wobei dieser Rest eine bis drei der folgenden Gruppen tragen kann: Halogen, Cyano, C₁-C₄-Alkoxy, C₁-C₄-Halogenalkoxy, C₁-C₄-Alkylthio und C₁-C₄-Alkoxycarbonyl,
C₃-C₇-Cycloalkyl oder Phenyl-(C₁-C₄)-alkyl, wobei die Ringe dieser Reste eine bis drei der folgenden Gruppen tragen können: Halogen, Cyano, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Alkoxycarbonyl, Aryl und Aryloxy;
- R⁴: Wasserstoff oder einen der Reste R³ oder
- R³ und R⁴,: gemeinsam mit dem C-Atom, an das sie gebunden sind, einen 4- bis 8-gliedrigen Ring, welcher als Ringglieder neben Kohlenstoff noch ein oder zwei der Heteroatome Sauerstoff, Schwefel und Stickstoff enthalten kann, wobei die Kohlenstoffatome im Ring eine oder zwei der folgenden Gruppen tragen können: Halogen, Cyano, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Alkoxycarbonyl, Aryl und Aryloxy und wobei Stickstoff als Heteroatom Wasserstoff oder eine C₁-C₄-Alkylgruppe trägt;
- R⁵: unabhängig von diesen einen der Reste R2;
- X: unabhängig voneinander Wasserstoff, C₁-C₈-Alkyl und/oder C₂-C₈-Alkenyl, wobei diese Reste partiell oder vollständig halogeniert sein und/oder eine bis drei der folgenden Gruppen tragen können: Cyano, C₁-C₄-Alkoxyalkyl, C₁-C₄-Halogenalkyl, C₁-C₄-Alkoxy, C₁-C₄-Halogenalkoxy, C₁-C₄-Alkylthio und C₁-C₄-Alkoxycarbonyl, Aryl und Aryloxy;
- Y: unabhängig voneinander und von diesen einen der Reste X;
- p,q: unabhängig voneinander 0, 1, 2;
- R⁶: Halogen, Cyano, Nitro, C₁-C₈-Alkyl, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Halogenalkylthio oder eine über Sauerstoff oder Schwefel gebundene Phenylgruppe, welche unsubstituiert ist oder einen bis drei der folgenden Substituenten tragen kann: Halogen, C₁-C₄-Alkyl und C₁-C₄-Alkoxy, wobei R⁶ für r > 1 für verschiedene der genannten Reste stehen kann;
- r: 0, 1, 2, 3.

Außerdem betrifft die Erfindung Verfahren zur Herstellung der Verbindungen I. Die Erfindung betrifft weiterhin Mittel, welche die Verbindungen I oder ihre Salze enthalten, ein Verfahren zur Herstellung derartiger Mittel sowie ein Verfahren zur Bekämpfung von Schadpilzen und die Verwendung der Verbindungen I, ihrer Salze oder der Mittel hierzu.

Verbindungen vom Typ I mit fungizider Wirkung sind bereits aus folgenden Druckschriften bekannt: vor allem EP-A 554 729 und DE-A 43 21 897, daneben EP-A 398 072, EP-A 425 925, EP-A 472 996, EP-A 477 639, EP-A 485 794, EP-A 493 683, EP-A 496 239, EP-A 550 788 und EP-A 587 110). Die bekannten Verbindungen befriedigen jedoch hinsichtlich ihrer fungiziden Wirkung noch nicht.

Der vorliegenden Erfindung lagen daher neue Carbamoylcarbonsäureamide mit verbesserter Wirkung gegen Schadpilze als Aufgabe zugrunde.

Demgemäß wurden die eingangs definierten Verbindungen I, ihre Salze, sowie sie enthaltende Mittel gefunden.

Ferner wurden Verfahren zur Herstellung der Verbindungen I und der sie enthaltenden Mittel gefunden und des weiteren ein Verfahren zur Bekämpfung von Schadpilzen und die Verwendung der Verbindungen I ihrer Salze oder der Mittel hierzu.

Der Gegenstand der zur vorliegenden Erfindung gehörigen deutschen Patentanmeldung P 44 31 467.1 ist hiermit einbezogen.

Die Verbindungen I können in an sich bekannter Weise ausgehend von den entsprechenden Carbamoylcarbonsäuren II hergestellt werden. Bevorzugt erhält man die Verbindungen I nach den im folgenden beschriebenen Verfahren A bzw. B (die Literaturzitate "Houben-Weyl" beziehen sich auf: Houben-Weyl, Methoden der Organischen Chemie, 4. Auflage, Thieme Verlag, Stuttgart).

### Verfahren A

Die Carbamoylcarbonsäureamide I erhält man, indem man die Carbamoylcarbonsäuren II mit den Aminen III umsetzt.

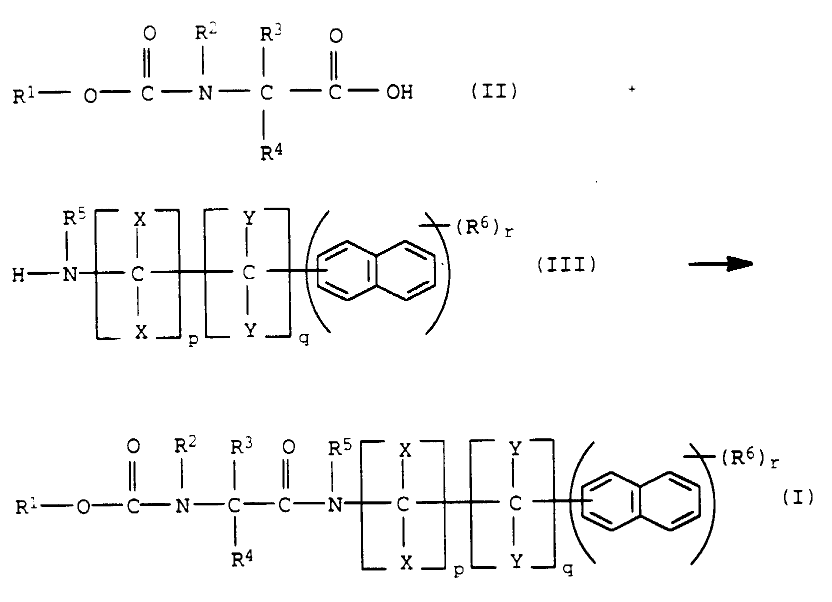

Die Carbamoylcarbonsäuren II sind bekannt oder können nach bekannten Methoden, vor allem ausgehend von den zugrundeliegenden Aminosäuren, hergestellt werden (vgl. "Houben-Weyl", Band 15/1, Seite 46 bis Seite 305, vor allem Seite 117 bis Seite 125).

Die Amine III sind ebenfalls bekannt oder können leicht erhalten werden (vgl. Organikum, VEB Deutscher Verlag der Wissenschaften, 15. Auflage, Berlin, 1977, Seite 610 ff.; "Houben-Weyl", Band 15/1, Seite 648-665; Indian J. Chem. 10, Seite 366 (1972)).

Vorzugsweise arbeitet man in diesem Verfahren A so, daß man zunächst die Carbamoylcarbonsäuren II in carboxyaktivierte Derivate, vor allem in Acylcyanide oder Anhydride, überführt (vgl. Tetrahedron Letters, Band 18, Seite 1595 bis Seite 1598 (1973), bzw. "Houben-Weyl", Band 15/1, Seite 28 bis Seite 32). Diese Derivate werden dann mit den Aminen III in Gegenwart von Basen zur Reaktion gebracht.

Zur Herstellung der carboxyaktivierten Acylcyanide eignet sich z.B. die Reaktion der Carbamoylcarbonsäuren II mit Cyanphosphonsäurediethylester, vor allem in einem inerten Lösungsmittel wie Tetrahydrofuran oder Toluol.

Zur Herstellung der carboxyaktivierten Anhydride ist die Umsetzung der Carbamoylcarbonsäure II mit Kohlensäurechloriden wie Chlorameisensäure-iso-butylester in Gegenwart von Basen und gegebenenfalls in einem inerten Lösungsmittel wie Toluol oder Tetrahydrofuran bevorzugt.

Die Umsetzung der Amine III mit den carboxyaktivierten Carbamoylcarbonsäuren II erfolgt vorzugsweise in einem Lösungsmittel wie Dichlormethan, Tetrahydrofuran oder Toluol.

Als Basen können insbesondere die Amine III selbst dienen, wobei man sie üblicherweise aus dem Rohprodukt zurückgewinnt.

In einer bevorzugten Ausführungsform dieser Verfahrensstufe werden die Carbamoylcarbonsäure II, das Amin III, das zur Erzeugung des carboxyaktivierten Derivates der Carbamoylcarbonsäure II geeignete Reagens und die Base im Eintopfverfahren, gegebenenfalls in einem inerten Lösungsmittel, zur Reaktion gebracht und das Rohprodukt anschließend in an sich bekannter Weise auf das Carbamoylcarbonsäureamid I aufgearbeitet.

### Verfahren B

Die Carbamoylcarbonsäureamide I, erhält man, indem man die Carbamoylcarbonsäureamide I, in denen die Gruppe R¹-O-(CO) für eine Schutzgruppe steht, die in an sich bekannter Weise abgespalten werden kann, in Aminosäureamide IV überführt und diese mit Chlorameisensäureestern V in Gegenwart einer Base umsetzt.

### Stufe Ba: Herstellung der Aminosäureamide IV

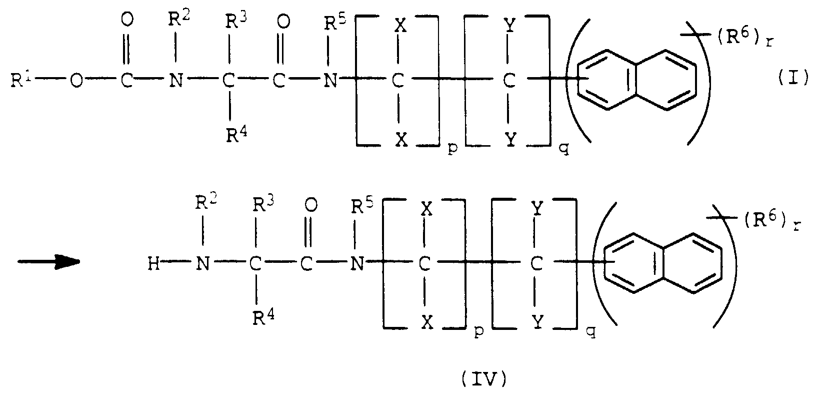

Die Abspaltung der Gruppe R¹-O-(CO) aus den Carbamoylcarbonsäureamiden I kann in an sich bekannter Weise durchgeführt werden (vgl. "Houben-Weyl", Band 15/1, Seite 46 bis Seite 305, vor allem Seite 126 bis Seite 129).

Geeignete abspaltbare Gruppen enthalten als Rest R¹ eine tert.-Butyl- oder die Benzylgruppe.

Im Falle von R¹ = tert.-Butyl beispielsweise erfolgt die Abspaltung üblicherweise durch Umsetzung mit einer Säure, insbesondere einer Protonensäure wie z.B. Salzsäure oder Trifluoressigsäure (ibid., Seite 126 bis Seite 129).

Die als Ausgangsstoffe geeigneten Carbamoylcarbonsäureamide I können nach bekannten Verfahren (vgl. "Houben-Weyl", Band 15/1, Seite 28 bis Seite 32) oder insbesondere nach dem erfindungsgemäßen Verfahren A gewonnen werden.

### Stufe Bb: Herstellung der Carbamoylcarbonsäureamide I

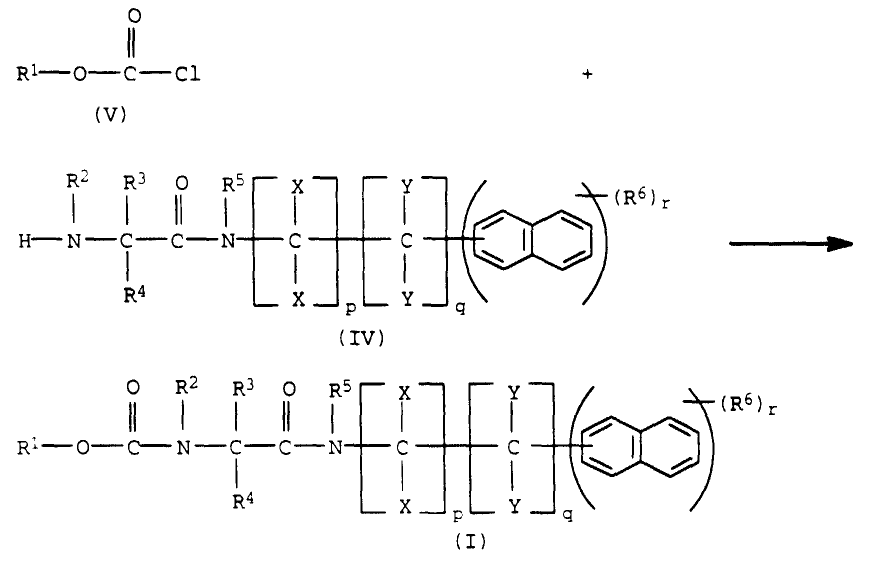

Die aus der Synthesestufe (Ba) resultierenden Aminosäureamide IV werden mit den Chlorameisensäureestern V in Gegenwart von Basen umgesetzt.

Die Chlorameisensäureester V sind allgemein bekannt oder können nach bekannten Verfahren hergestellt werden.

Die Umsetzung wird vorzugsweise in einem organischen Lösungsmittel, vor allem Toluol, Methylenchlorid oder Tetrahydrofuran, oder Gemischen dieser Lösungsmittel durchgeführt.

Als Basen kommen anorganische und organische Basen gleichermaßen in Betracht, wobei organische Basen und hierunter wiederum tertiäre Amine wie Triethylamin, Pyridin und N-Methylpiperidin bevorzugt sind.

Die Umsetzung wird in der Regel bei Temperaturen von (-40) bis 50, vorzugsweise (-10) bis 20°C durchgeführt.

Im übrigen ist die Durchführung dieser Reaktion dem Fachmann geläufig,.so daß es keiner weiteren Ausführungen hierzu bedarf (vgl. "Houben-Weyl", Band 15/1, Seite 117 bis Seite 139).

Die nach den Verfahren A bzw. B erhaltenen Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit werden können. Sofern die Zwischen- und Endprodukte als Feststoffe erhalten werden, kann die Reinigung auch beispielsweise durch Umkristallisieren oder Digerieren erfolgen.

Die Verbindungen der Formel I können gegebenenfalls in Abhängigkeit von der Art der Substituenten als geometrische und/oder optische Isomere oder Isomerengemische anfallen. Sowohl die reinen Isomeren als auch die Gemische der Isomeren weisen die fungizide Wirkung auf.

Teil der Erfindung sind auch die Salze vor allem der säurebeständigen Verbindungen I, welche basische Zentren, vor allem basische Stickstoffatome enthalten, insbesondere mit Mineralsäuren wie Schwefelsäure und Phosphorsäure oder Lewis-Säuren wie Zinkchlorid. Üblicherweise kommt es hierbei auf die Art des Salzes nicht an. Im Sinne der Erfindung sind solche Salze bevorzugt, die die von Schadpilzen freizuhaltenden Pflanzen, Flächen, Materialien oder Räume nicht schädigen und die Wirkung der Verbindungen I nicht beeinträchtigen. Besonders bedeutsam sind derartige landwirtschaftlich brauchbare Salze.

Die Salze der Verbindungen I sind in an sich bekannter Weise zugänglich, vor allem durch Umsetzen der entsprechenden Carbamoylcarbonsäureamide I mit den genannten Säuren in Wasser oder einem inerten organischen Lösungsmittel bei Temperaturen von (-80) bis 120°C, vorzugsweise 0 bis 60°C.

Die Herstellung der Salze ist in der Regel vom Druck unabhängig, weshalb man vor allem bei Atmosphärendruck arbeitet.

Bei der eingangs angegebenen Definition der Verbindungen I wurden Sammelbegriffe verwendet, die repräsentativ für die folgenden Substituenten stehen:
Halogen: Fluor, Chlor, Brom und Jod;
Alkyl: geradkettige oder verzweigte Alkylgruppen mit 1 bis 8 Kohlenstoffatomen, z.B. C₁-C₆-Alkyl wie Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 2,2-Dimethylpropyl, 1,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 2,2-Dimethylbutyl, 3,3-Dimethylbutyl, 1,2-Dimethylbutyl, 1,3-Dimethylbutyl, 2,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Halogenalkyl bzw. partiell oder vollständig halogeniertes Alkyl: geradkettige oder verzweigte Alkylgruppen mit 1 bis 4 bzw. 8 Kohlenstoffatomen (wie vorstehend genannt), wobei in diesen Gruppen die Wasserstoffatome partiell oder vollständig durch Halogenatome (wie vorstehend genannt) ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkoxygruppen mit 1 bis 4 Kohlenstoffatomen, z.B. C₁-C₃-Alkoxy wie Methyloxy, Ethyloxy, Propyloxy und 1-Methylethyloxy;
Alkoxyalkyl: geradkettige oder verzweigte Alkylgruppen mit 1 bis 8 Kohlenstoffatomen (wie vorstehend genannt), welche in einer beliebigen Position eine geradkettige oder verzweigte Alkoxygruppe (wie vorstehend genannt) mit im Falle von C₁-C₄-Alkoxyalkyl 1 bis 4 Kohlenstoffatomen tragen, wie Methoxymethyl, Ethoxymethyl, n-Propoxymethyl, n-Butoxymethyl, 1-Methoxyethyl, 2-Methoxyethyl, 1-Ethoxyethyl, 2-Ethoxyethyl, 2-n-Propoxyethyl und 2-Butoxyethyl;
Halogenalkoxy: geradkettige oder verzweigte Alkoxygruppen mit 1 bis 4 Kohlenstoffatomen (wie vorstehend genannt), wobei in diesen Gruppen die Wasserstoffatome partiell oder vollständig durch Halogenatome (wie vorstehend genannt) ersetzt sein können, z.B. C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy,, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
Alkylthio: geradkettige oder verzweigte Alkylgruppen mit 1 bis 4 Kohlenstoffatomen (wie vorstehend genannt), welche über ein Schwefelatom (-S-) an das Gerüst gebunden sind, z.B. C₁-C₄-Alkylthio wie Methylthio, Ethylthio, Propylthio, 1-Methylethylthio, n-Butylthio und tert.-Butylthio;
Alkoxycarbonyl: geradkettige oder verzweigte Alkoxygruppen mit 1 bis 4 C-Atomen (wie vorstehend genannt), welche über eine Carbonylgruppe (-CO-) an das Gerüst gebunden sind;
Alkenyl: geradkettige oder verzweigte Alkenylgruppen mit 2 bis 8 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 2-Methyl-1-propenyl, 1-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimetnyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-l-butenyl, 1,3-Dimethyl-2-butenyl, 1,3-Dimethyl-3-butenyl, 2,2-Dimethyl-3butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
Alkinyl: geradkettige oder verzweigte Alkinylgruppen mit 2 bis 8 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-l-butinyl, 1-Ethyl-2-butinyl, 1-Ethyl-3-butinyl, 2-Ethyl-3-butinyl und 1-Ethyl-1-methyl-2-propinyl;
Cycloalkyl: monocyclische Alkylgruppen mit 3 bis 7 Kohlenstoffringgliedern, z.B. C₃-C₇-Cycloalkyl wie Cyclopropyl, Cyclobutyl, Cyclopentyl, Cyclohexyl und Cycloheptyl;
Cycloalkenyl: monocyclische Alkylgruppen mit 5 bis 7 Kohlenstoffringgliedern die eine oder mehrere Doppelbindungen enthalten z.B. C₅-C₇-Cycloalkenyl wie Cyclopentenyl, Cyclohexenyl und Cycloheptenyl;
nicht-aromatische 4- bis 8-gliedrigen Ringe, welcher als Ringglieder neben Kohlenstoff noch ein oder zwei Sauerstoff-, Schwefel- oder Stickstoffatome enthalten wie gesättigte 5- oder 6-gliedrige Ringe mit 1 oder 2 Stickstoff- und/oder Sauerstoffatomen wie 3-Tetrahydrofuranyl, 1-Piperidinyl, 2-Piperidinyl, 3-Piperidinyl, 4-Piperidinyl, 2-Tetrahydropyranyl, 3-Tetrahydropyranyl, 4-Tetrahydropyranyl, 2-Morpholinyl und 3-Morpholinyl;
Aryl: monocyclische oder polycyclische aromatische Gruppen mit 6 bis 10 C-Atomen wie Phenyl und Naphthyl;
Arylalkyl: Arylgruppen (wie vorstehend genannt), welche im Falle von Aryl-(C₁-C₄)-alkyl über Alkylgruppen mit 1 bis 4 Kohlenstoffatomen (wie vorstehend genannt) an das Gerüst gebunden sind, z.B. Phenyl-(C₁-C₄)-alkyl wie Benzyl, 2-Phenylethyl, 3-Phenylpropyl, 4-Phenylbutyl, 1-Phenylethyl, 1-Phenylpropyl und 1-Phenylbutyl;
Aryloxy: Arylgruppen (wie vorstehend genannt), welche über ein Sauerstoffatom (-O-) an das Gerüst gebunden sind wie Phenoxy, 1-Naphthoxy und 2-Naphthoxy;
Heteroaryl: aromatische mono- oder polycyclische Reste, welche neben Kohlenstoffringgliedern zusätzlich 1 bis 4 Stickstoffatome oder 1 bis 3 Stickstoffatome und ein Sauerstoff- oder ein Schwefelatom oder ein Sauerstoff- oder ein Schwefelatom enthalten können, z.B.:
- 5-gliedriges Heteroaryl, enthaltend 1 bis 3 Stickstoffatome: 5-Ring-Heteroarylgruppen, welche neben Kohlenstoffatomen 1 bis 3 Stickstoffatome als Ringglieder enthalten können, z.B. 2-Pyrrolyl, 3-Pyrrolyl, 3-Pyrazolyl, 4-Pyrazolyl, 5-Pyrazolyl, 2-Imidazolyl, 4-Imidazolyl, 1,2,4-Triazol-3-yl und 1,3,4-Triazol-2-yl;
- 5-gliedriges Heteroaryl, enthaltend 1 bis 4 Stickstoffatome oder 1 bis 3 Stickstoffatome und 1 Schwefelatom oder Sauerstoffatom oder 1 Sauerstoff- oder 1 Schwefelatom: 5-Ring-Heteroarylgruppen, welche neben Kohlenstoffatomen 1 bis 4 Stickstoffatome oder 1 bis 3 Stickstoffatome und 1 Schwefel- oder Sauerstoffatom oder 1 Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl;
- benzokondensiertes 5-gliedriges Heteroaryl, enthaltend 1 bis 3 Stickstoffatome oder 1 Stickstoffatom und/oder ein Sauerstoff- oder Schwefelatom: 5-Ring-Heteroarylgruppen, welche neben Kohlenstoffatomen 1 bis 4 Stickstoffatome oder 1 bis 3 Stickstoffatome und 1 Schwefel- oder Sauerstoffatom oder 1 Sauerstoff- oder ein Schwefelatom als Ringglieder enthalten können, und in welchen 2 benachbarte Kohlenstoffringglieder oder 1 Stickstoff- und 1 benachbartes Kohlenstoffringglied durch eine Buta-1,3-dien-1,4-diylgruppe verbrückt sein können;
- über Stickstoff gebundenes 5-gliedriges Heteroaryl, enthaltend 1 bis 4 Stickstoffatome, oder über Stickstoff gebundenes benzokondensiertes 5-gliedriges Heteroaryl, enthaltend 1 bis 3 Stickstoffatome: 5-Ring-Heteroarylgruppen, welche neben Kohlenstoffatomen 1 bis 4 Stickstoffatome bzw. 1 bis 3 Stickstoffatome als Ringglieder enthalten können, und in welchen 2 benachbarte Kohlenstoffringglieder oder ein Stickstoff- und ein benachbartes Kohlenstoffringglied durch eine Buta-1,3-dien-1,4-diylgruppe verbrückt sein können, wobei diese Ringe über eines der Stickstoffringglieder an das Gerüst gebunden sind;
- 6-gliedriges Heteroaryl, enthaltend 1 bis 3 bzw. 1 bis 4 Stickstoffatome: 6-Ring-Heteroarylgruppen, welche neben Kohlenstoffatomen 1 bis 3 bzw. 1 bis 4 Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
- benzokondensiertes 6-gliedriges Heteroaryl, enthaltend 1 bis 4 Stickstoffatome: 6-Ring-Heteroarylgruppen, in welchen 2 benachbarte Kohlenstoffringglieder durch eine Buta-1,3-dien-1,4-diylgruppe verbrückt sein können, z.B. Chinolin, Isochinolin, Chinazolin und Chinoxalin.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome, wie vorstehend genannt, ersetzt sein können.

Im Hinblick auf ihre Wirkung gegen Schadpilze sind Verbindungen I bevorzugt, in denen die Reste die folgenden Bedeutungen haben, und zwar für sich allein oder in Kombination. Die in den Restebedeutungen im folgenden genannten Gruppen können auch anspruchsgemäß substituiert sein.
- R¹: C₁-C₈-Alkyl und vor allem C₁-C₄-Alkyl;
- R²: Wasserstoff oder C₁-C₈-Alkyl;
- R³: C₁-C₈-Alkyl, C₃-C₇-Cycloalkyl oder Phenyl-(C₁-C₄)-alkyl, vor allem C₁-C₈-Alkyl und insbesondere C₃-Alkyl, vorzugsweise iso-Propyl;
- R⁴: C₁-C₈-Alkyl und vor allem Wasserstoff;
- R⁵: Wasserstoff;
- X: unabhängig voneinander Wasserstoff oder C₁-C₈-Alkyl, vorzugsweise C₁-C₈-Alkyl, vor allem C₁-C₄-Alkyl;
- Y: unabhängig voneinander Wasserstoff oder C₁-C₈-Alkyl, vor allem Wasserstoff;
- p: 0 oder 1, vor allem 1
- q: 0 oder 1, vor allem 0;
- R⁶: Halogen, C₁-C₈-Alkyl, vor allem Halogen oder C₁-C₄-Alkyl;
- r: 0 oder 1, vor allem 0.

Ganz besonders bevorzugt sind im Hinblick auf ihre Verwendung die in den anschließenden Tabellen 1 bis 5 zusammengestellten Verbindungen I.

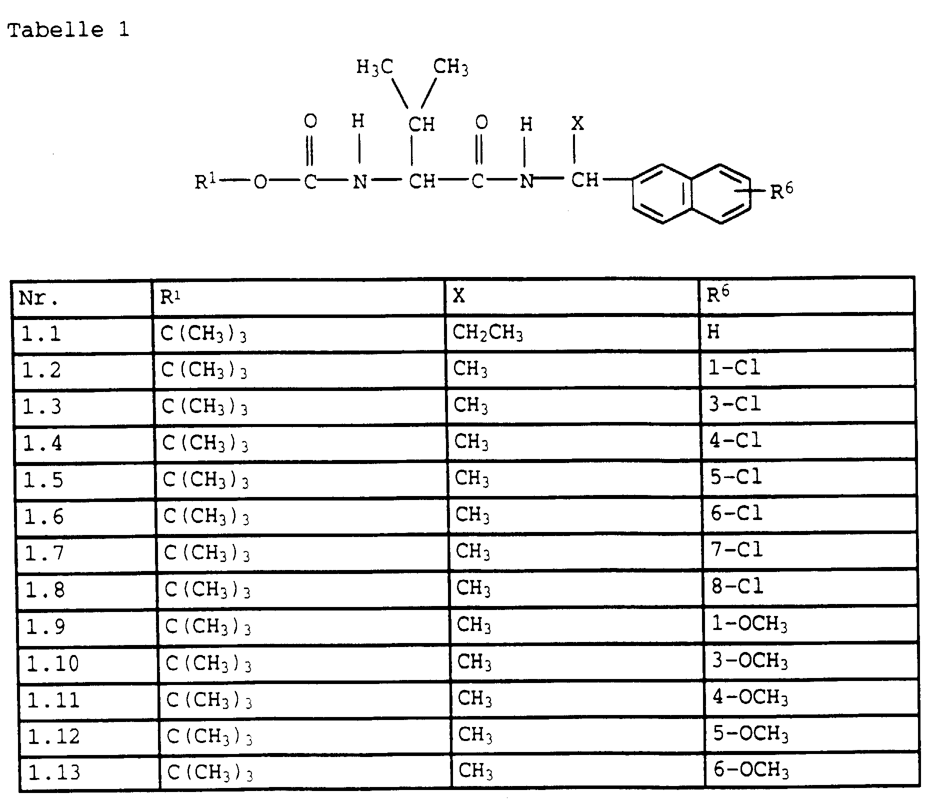

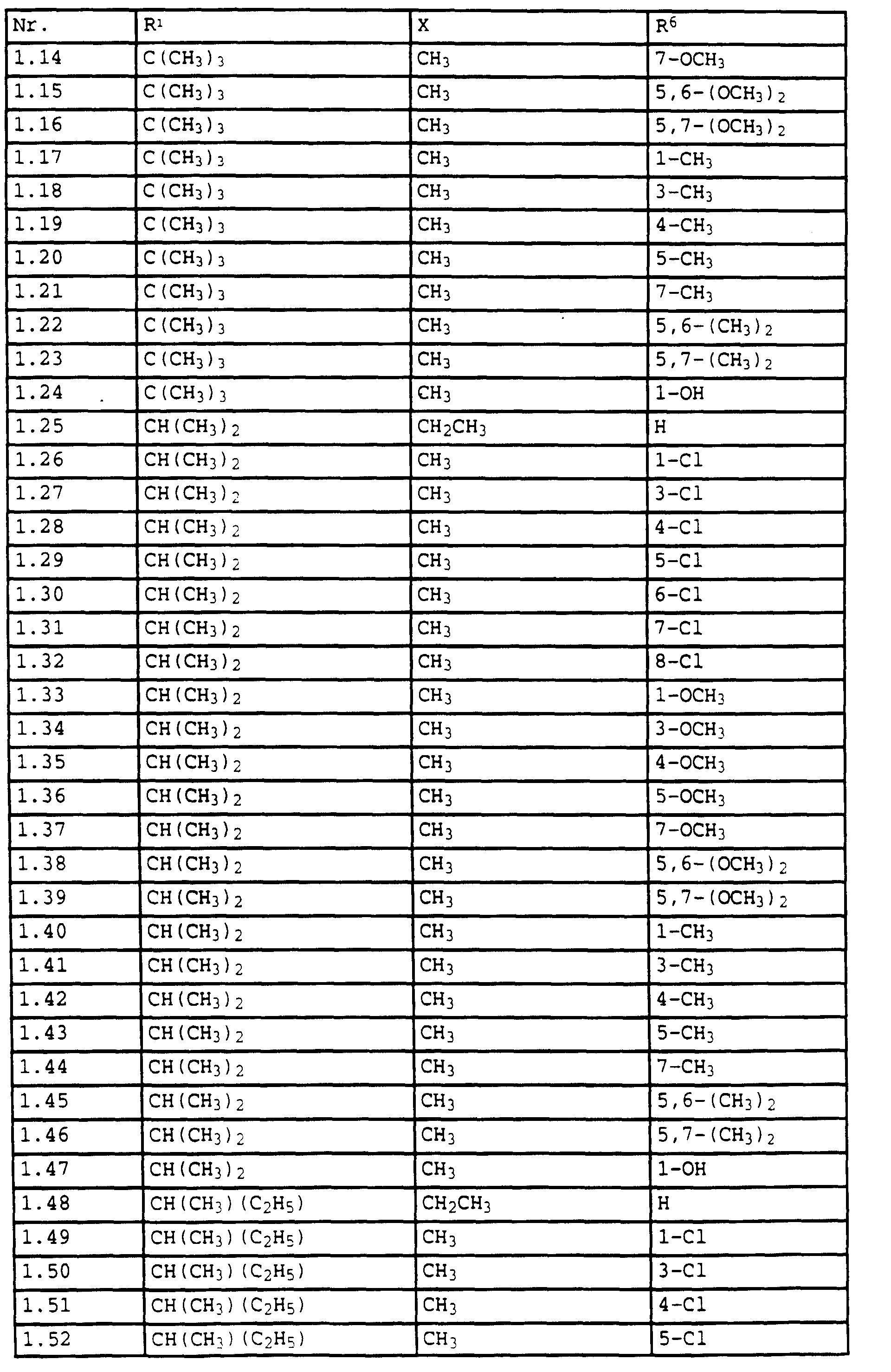

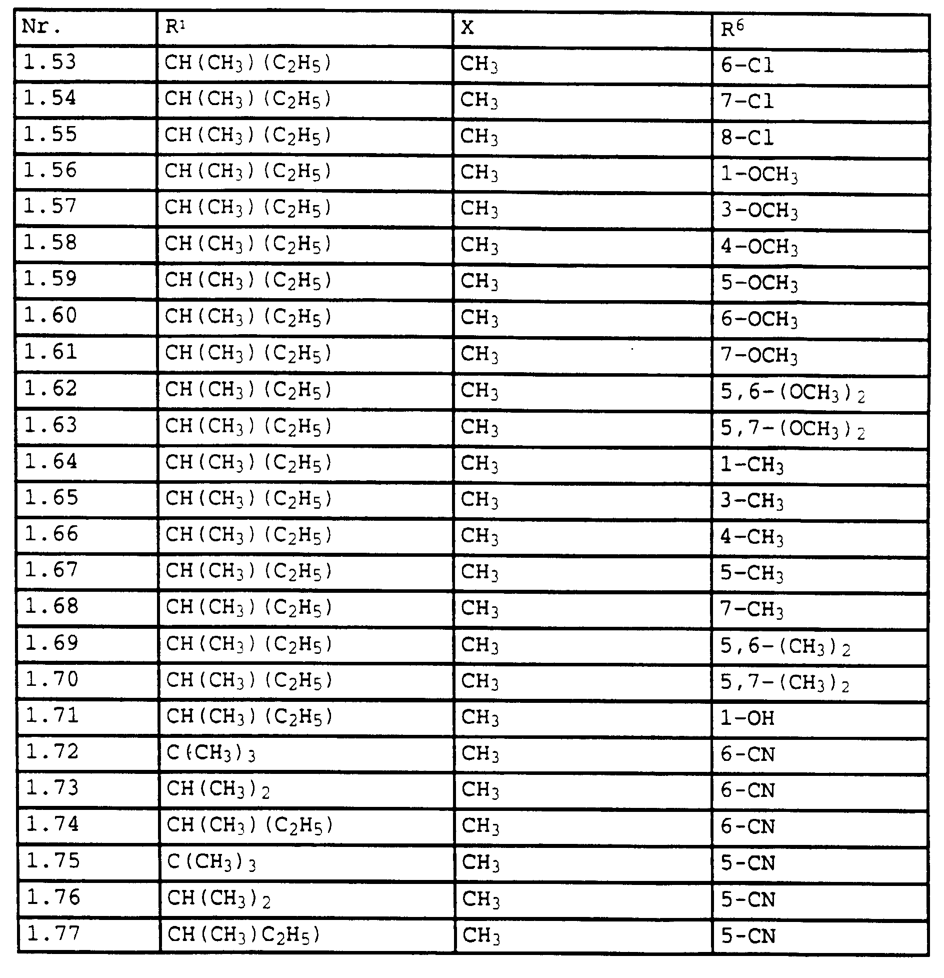

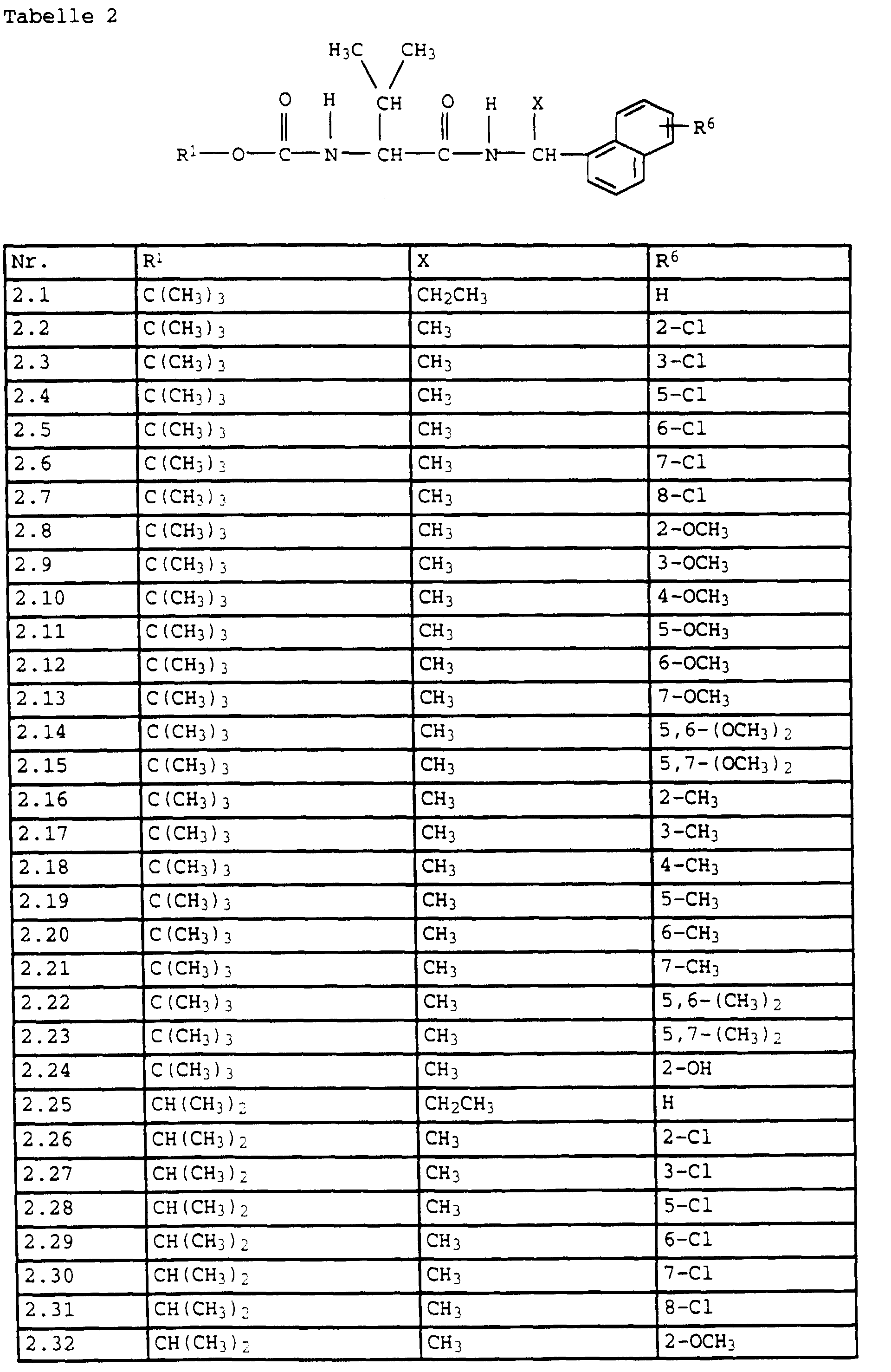

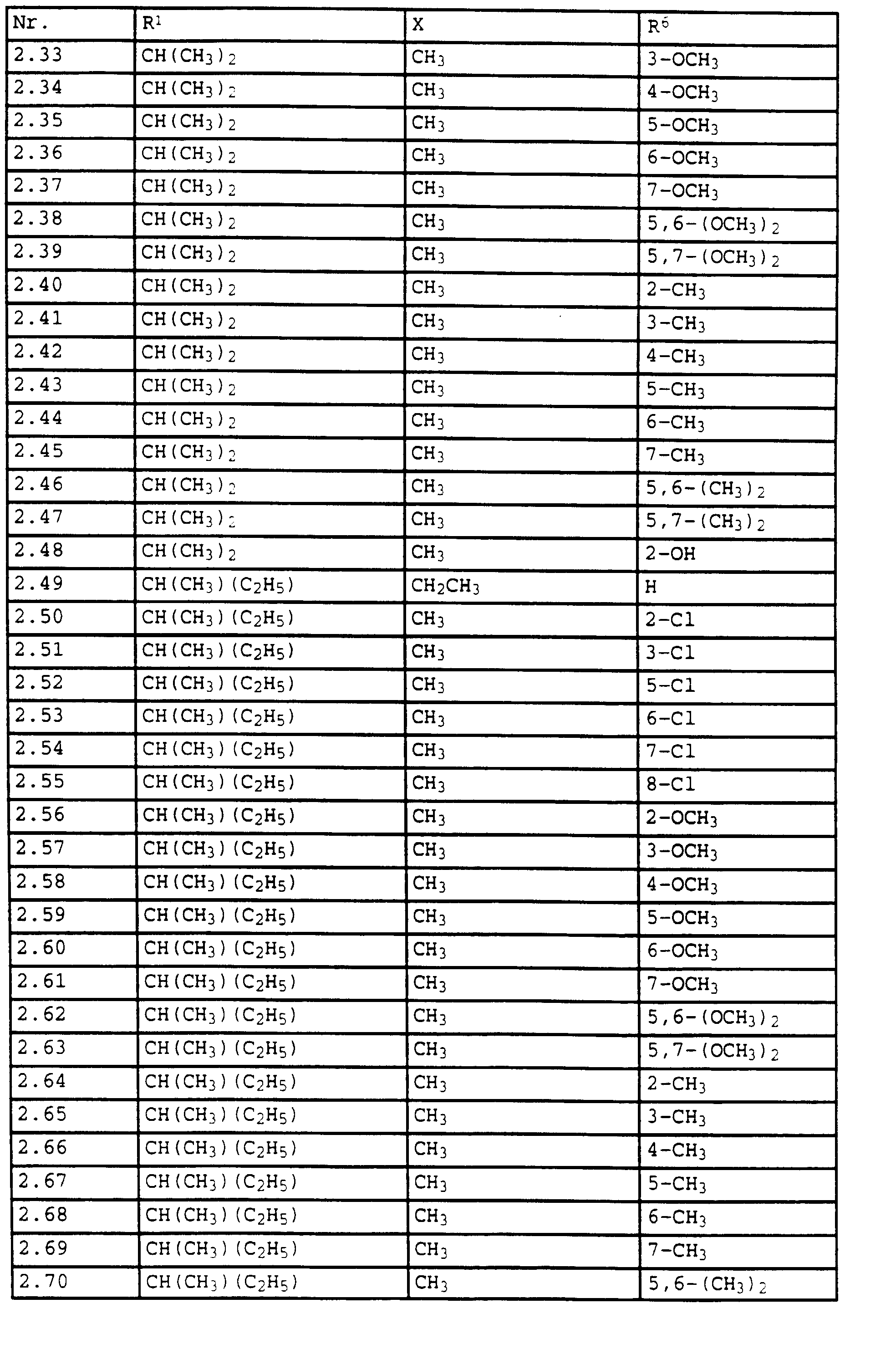

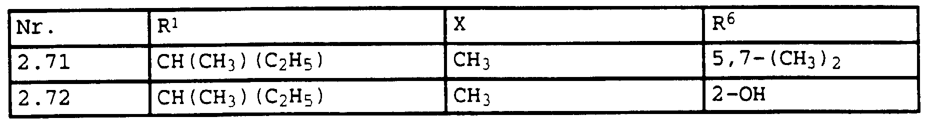

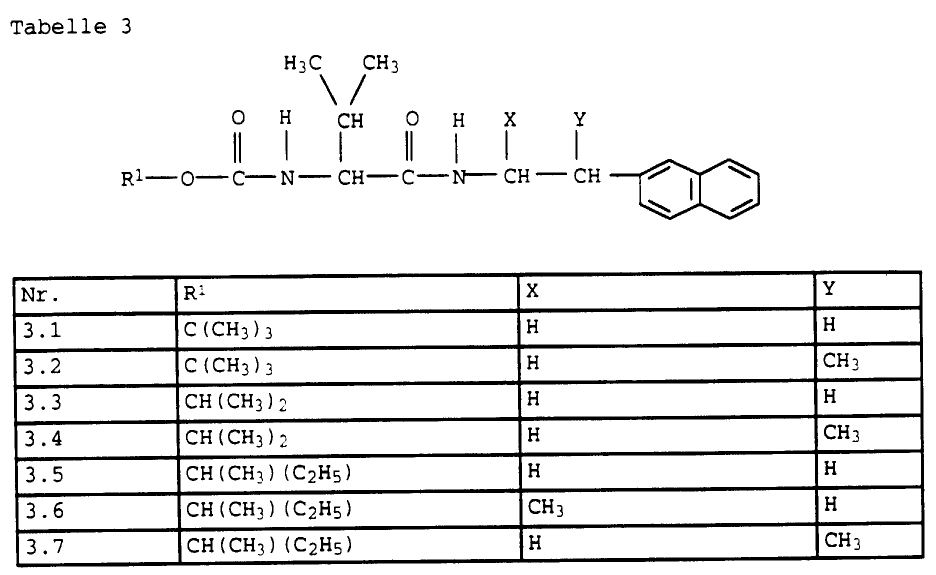

Die neuen Verbindungen der Formel I und deren Salze eignen sich zur Bekämpfung von Schadpilzen.

Die neuen Verbindungen I oder ihre Salze können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Normalerweise werden bei der Pflanzenbehandlung die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes iso-Octyl-, Octyl- oder Nonylphenol, Alkylphenol-, Tributylphenylpolyglykolether, Alkylarylpolyetheralkohole, iso-Tridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
I. eine Lösung aus 90 Gew.-Teilen einer erfindungsgemäßen Verbindung I und 10 Gew.-Teilen N-Methyl-2-pyrrolidon, die zur Anwendung in Form kleinster Tropfen geeignet ist;
II. eine Mischung aus 10 Gew.-Teilen einer erfindungsgemäßen Verbindung I, 70 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 10 Gew.-Teilen einer erfindungsgemäßen Verbindung I, 40 Gew.-Teilen Cyclohexanon, 30 Gew.-Teilen iso-Butanol, 20 Gew.-Teilen des Anlagerungsproduktes von 40 mol Ethylenoxid an 1 mol Ricinusöl;
IV. eine wäßrige Dispersion aus 10 Gew.-Teilen einer erfindungsgemäßen Verbindung I, 25 Gew.-Teilen Cyclohexanol, 55 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen, vorzugsweise einer festen erfindungsgemäßen Verbindung I, 3 Gew.-Teilen des Natriumsalzes der Di-isobutylnaphthalin-2-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 62 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenolsulfonsäure-Harnstoff-Formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkoholpolyglykolether, 20 Gew.-Teilen des Natriumsalzes eines Phenolsulfonsäure-Harnstoff-Formaldehyd-Kondensates und 50 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Deuteromyceten, Ascomyceten, Phycomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die neuen Verbindungen zur Bekämpfung folgender Pflanzenkrankheiten:

Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Zierpflanzen und Gemüse, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,025 und 2, vorzugsweise 0,1 bis 1 kg Wirkstoff pro ha.

Bei der Saatgutbehandlung werden im allgemeinen Wirkstoffmengen von 0,001 bis 50, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfid, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)-disulfid;
Nitroderivate, wie Dinitro-(1-methylheptyl)-phenylcrotonat, 2-sec.-Butyl-4,6-dinitrophenyl-3,3-dimethylacrylat, 2-sec.-Butyl-4,6-dinitrophenyl-iso-propylcarbonat, 5-Nitro-iso-phthalsäure-di-iso-propylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4-triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thion-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethylfuran-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan,
2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl] -cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, (2-Chlorphenyl)-(4-chlorphenyl)-5-pyrimidin-methanol, 5-Butyl-2-dimethylamino-4-hydroxy-6-methyl-pyrimidin, Bis- (p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol, [2-(4-Chlorphenyl)ethyl]-(1,1-dimethylethyl)-1H-1,2,4-triazol-1-ethanol, 1-[3-(2-Chlorphenyl)-1-(4-fluorphenyl)oxiran-2-yl-methyl]-1H-1,2,4-triazol sowie
verschiedene Fungizide, wie Dodecylguanidinacetat, 3-[3-(3,5-Dimethyl-2-oxycyclohexyl)-2-hydroxyethyl]glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[(3,5-Dichlorphenyl)-5-methyl-5-methoxymethyl]-1,3-oxazolidin-2,4-dion, 3-(3,5-Dichlorphenyl)-1-iso-propylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl) -1H-1,2,4-triazol,
Strobilurine wie Methyl-E-methoximino-[α-(o-tolyloxy)-o-tolyl]acetat, Methyl-E-2-{2-[6-(2-cyanophenoxy)pyridimin-4-yloxy]phenyl}-3-methoxyacrylat, Methyl-E-methoximino-[a-(2,5-dimethyloxy)-o-tolyl]acetamid.

Anilino-Pyrimidine wie N-(4,6-dimethylpyrimidin-2-yl)anilin, N-[4-methyl-6-(1-propinyl)pyrimidin-2-yl] anilin, N-(4-methyl-6-cyclopropyl-pyrimidin-2-yl)anilin.

Phenylpyrrole wie 4-(2,2-difluor-1,3-benzodioxol-4-yl)-pyrrol-3-carbonitril.

Zimtsäureamide wie 3-(4-chlorphenyl)-3-(3,4-dimethoxyphenyl)acrylsäuremorpholid.

### Synthesebeispiel

Die im nachstehenden Synthesebeispiel wiedergegebene Vorschrift kann unter Abwandlung der Ausgangsverbindung zur Gewinnung weiterer Vertreter der Verbindungen I benutzt werden. Die physikalischen Daten der demgemäß hergestellten Produkte sind in den Tabellen 4 und 5 wiedergegeben.

### N-(iso-Propyloxycarbonyl)-L-valin-(1-(β-naphthyl)-ethyl)-amid (Verbindung Nr. 4.2 in Tabelle 4)

Zu einer Lösung von 14,2 g (65 mmol) tert.-Butoxycarbonyl-L-valin und 13,6 g (65 mmol) l-Amino-1-(β-naphthyl)-ethan in 300 ml Tetrahydrofuran wurden 13,3 g (65 mmol) Cyanphosphorsäurediethylester gegeben. Es wurde eine Stunde bei 0°C und 15 Stunden bei 20°C nachgerührt. Anschließend wurde das Lösungsmittel entfernt und der Rückstand mit 300 ml Essigsäureethylester aufgenommen. Die organische Phase wurde mit jeweils 200 ml 5 gew.-%iger Natronlauge, 10 %iger Salzsäure, 10 gew.-%iger Natriumhydrogencarbonatlösung und Wasser gewaschen, getrocknet und eingeengt. Es verblieben 18,0 g (49 mmol) N-(tert.-Butyloxycarbonyl)-L-valin-(1-(β-naphthyl)-ethyl)-amid (Fp. 97°C).

Zu 17,0 g (46 mmol) dieser Verbindung wurden unter Kühlung 50 ml Trifluoressigsäure gegeben und die Mischung 1 Stunde bei 0°C gerührt. Anschließend wurde auf 20°C erwärmt, die Trifluoressigsäure weitgehend abdestilliert, der Rückstand in 300 ml Dichlormethan aufgenommen und dieser mit je 200 ml 2 N Natronlauge, 5 gew.-%iger Natriumhydrogencarbonatlösung sowie Wasser gewaschen. Nach Trocknen und Einengen der organischen Phase verblieben 10,7 g (40 mmol) L-Valin-(1-(β-naphthyl)-ethyl)-amid als gelbes zähflüssiges Öl.

0,54 g (2,0 mmol) dieser Verbindung und 0,22 g (2,2 mmol) Triethylamin in 40 ml Toluol wurden bei 0°C mit 0,24 g (2,1 mmol) Chlorameisensäure-iso-propylester versetzt und 15 Stunden bei 20°C gerührt. Nach Entfernen des Lösungsmittels wurde der Rückstand mit 50 ml Essigsäureethylester aufgenommen und mit jeweils 40 ml 10 gew.-%iger Salzsäure, 10 gew.-%iger Natriumhydrogencarbonatlösung und Wasser gewaschen. Nach dem Trocknen der organischen Phase wurde das Lösungsmittel entfernt. Es verblieben 0,58 g (1,6 mmol) der Titelverbindung als farbloser kristalliner Rückstand (Fp. 127°C).

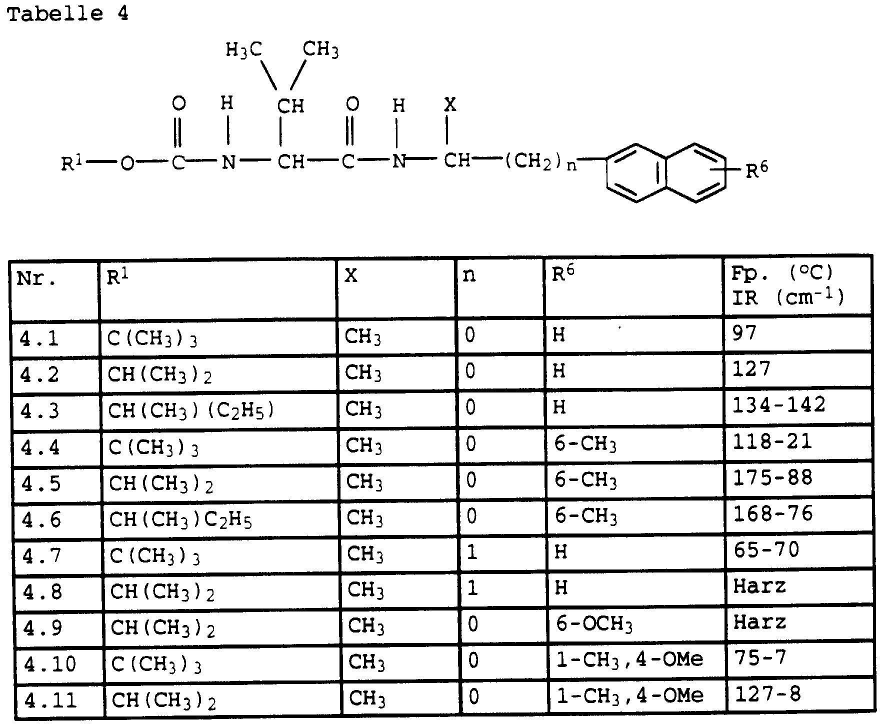

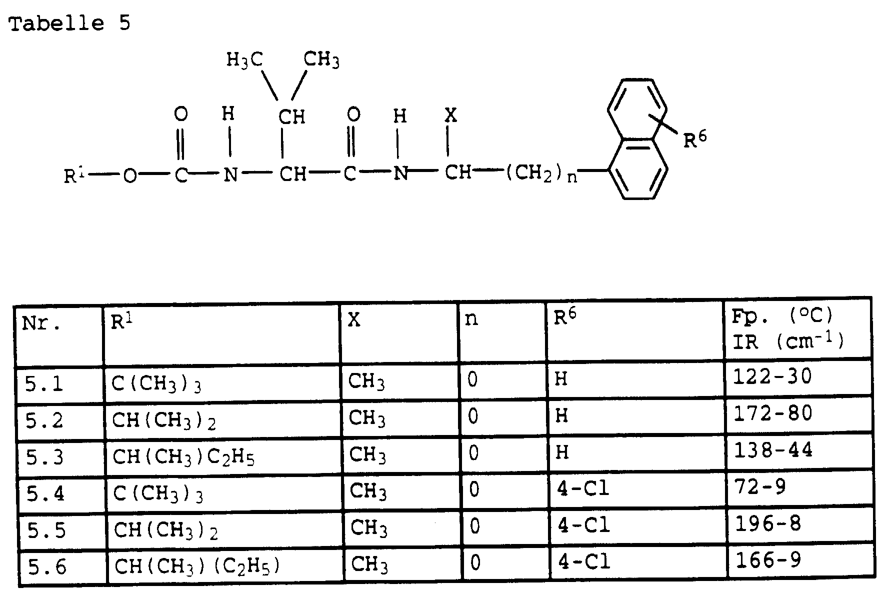

### Anwendungsbeispiele

Für die folgenden Versuche, welche die fungizide Wirkung der Verbindungen I zeigen sollen, wurde eine Emulsion verwendet, welche zu 10 Gew.-% aus dem Wirkstoff, und zu 80 Gew.-% aus einem Gemisch aus
- 70 Gew.-% Cyclohexanol,
- 20 Gew.-% Nekanil® LN (Lutensol® AP6, Netzmittel mit Emulgier- und Dispergierwirkung auf der Basis ethoxylierter Alkylphenole) und
- 10 Gew.-% Emulphor® EL (Emulan© EL, Emulgator auf der Basis ethoxylierter Fettalkohole)
bestand. Die gewünschte Wirkstoff-Konzentration wurde durch Verdünnen dieser Emulsion mit Wasser eingestellt.

### Plasmopara viticola

Blätter von Topfreben der Sorte "Müller-Thurgau" wurden mit wäßriger Spritzbrühe, die 80 Gew.-% Wirkstoff und 20 Gew.-% Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Die Reben wurden zunächst für 48 Stunden in einer Kammer mit wasserdampfgesättigter Luft bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die visuelle Beurteilung des Ausmaßes des Pilzausbruchs auf den Blattunterseiten.

In diesem Test zeigten die Blätter von Pflanzen, die mit einer 250 ppm oder 63 ppm enthaltenden wäßrigen Aufbereitung jeweils einer der Verbindungen Nr. 4.1, 4.2, 4.3, 4.4, 4.5, 4.6, 4.7, 4.8, 4.9, 4.10, 5.2, 5.3, 5.4, 5.5 und 5.6 behandelt worden waren, im Falle von 250 ppm Wirkstoff einen Pilzbefall auf 0 bis 5 % der Blattfläche und bei 63 ppm auf 0 bis 25 % der Blattfläche. Die Blätter der unbehandelten Pflanzen waren hingegen zu 80 % befallen.

### Phytophthora infestans

Blätter von Topfpflanzen der Sorte "Große Fleischtomate" wurden mit wäßriger Spritzbrühe, die 80 Gew.-% Wirkstoff und 20 Gew.-% Emulgiermittel in der Trockensubstanz enthielt, besprüht. Nach 24 Stunden wurden die Blätter mit einer Zoosporenaufschwemmung des Pilzes Phytophthora infestans infiziert. Die Pflanzen wurden dann in einer Kammer mit wasserdampfgesättigter Luft bei Temperaturen zwischen 16 und 18°C aufgestellt. Nach 6 Tagen hatte sich der Befall auf den unbehandelten, jedoch infizierten Kontrollpflanzen so stark entwickelt, daß die fungizide Wirksamkeit der Substanzen visuell beurteilt werden konnte.

In diesem Test zeigten die Blätter von Pflanzen, die mit einer 250 ppm oder 63 ppm enthaltenden wäßrigen Aufbereitung jeweils einer der Verbindungen Nr. 4.1, 4,2, 4.3, 4.4, 4.5, 4.6, 4.8, 4.9, 5.2, 5.4, 5.5 und 5.6 behandelt worden waren, einen Pilzbefall auf 0 bis 15 % der Blattfläche. Die Blätter der unbehandelten Pflanzen waren hingegen zu 90 % befallen.

## Patentansprüche

1. Carbamoylcarbonsäureamide der allgemeinen Formel I 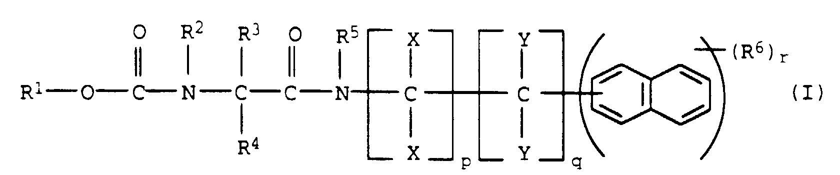 sowie deren Salze, in denen die Variablen die folgende Bedeutung haben:
R¹ C₁-C₈-Alkyl, C₂-C₈-Alkenyl oder C₂-C₈-Alkinyl, wobei diese Reste partiell oder vollständig halogeniert sein und/oder eine bis drei der folgenden Gruppen tragen können: Cyano, C₁-C₄-Alkoxy, C₁-C₄-Halogenalkoxy, C₁-C₄-Alkylthio, C₁-C₄-Alkoxycarbonyl, C₃-C₇-Cycloalkyl, C₃-C₇-Cycloalkenyl, Aryl, Aryloxy und Heteroaryl, wobei die cyclischen und aromatischen Ringe dieser Gruppen ihrerseits einen bis drei der folgenden Substituenten tragen können: Halogen, Cyano, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Alkoxycarbonyl, Aryl, Aryloxy und Heteroaryl,
C₃-C₇-Cycloalkyl oder C₃-C₇-Cycloalkenyl, wobei diese Reste partiell oder vollständig halogeniert sein und/oder eine bis drei der folgenden Gruppen tragen können: Cyano, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Alkoxycarbonyl, Aryl, Aryloxy und Aryl-(C₁-C₄)-alkyl, wobei die aromatischen Ringe dieser Gruppen ihrerseits einen bis drei der folgenden Substituenten tragen können: Halogen, Cyano, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Alkoxycarbonyl, Aryl und Aryloxy,
einen nicht-aromatischen 4- bis 8-gliedrigen Ring, welcher als Ringglieder neben Kohlenstoff noch eines oder zwei der Heteroatome Sauerstoff, Schwefel und Stickstoff enthalten kann, wobei die Kohlenstoffatome im Ring eine oder zwei der folgenden Gruppen tragen können: Halogen, Cyano, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Alkoxycarbonyl, Aryl und Aryloxy und wobei das zweite und jedes weitere Stickstoffatom als Heteroatom im Ring Wasserstoff oder eine C₁-C₄-Alkylgruppe trägt;
R² Wasserstoff, C₁-C₈-Alkyl oder C₃-C₇-Cycloalkyl, welche partiell oder vollständig halogeniert sein können;
R³ C₁-C₈-Alkyl, wobei dieser Rest eine bis drei der folgenden Gruppen tragen kann: Halogen, Cyano, C₁-C₄-Alkoxy, C₁-C₄-Halogenalkoxy, C₁-C₄-Alkylthio und C₁-C₄-Alkoxycarbonyl,
C₃-C₇-Cycloalkyl oder Phenyl-(C₁-C₄)-alkyl, wobei die Ringe dieser Reste eine bis drei der folgenden Gruppen tragen können: Halogen, Cyano, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Alkoxycarbonyl, Aryl und Aryloxy;
R⁴ Wasserstoff oder einen der Reste R³ oder
R³ und R⁴, gemeinsam mit dem C-Atom, an das sie gebunden sind, einen 4- bis 8-gliedrigen Ring, welcher als Ringglieder neben Kohlenstoff noch ein oder zwei der Heteroatome Sauerstoff, Schwefel und Stickstoff enthalten kann, wobei die Kohlenstoffatome im Ring eine oder zwei der folgenden Gruppen tragen können: Halogen, Cyano, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Alkoxycarbonyl, Aryl und Aryloxy und wobei Stickstoff als Heteroatom Wasserstoff oder eine C₁-C₄-Alkylgruppe trägt;
R⁵ unabhängig von diesen einen der Reste R²;
X unabhängig voneinander Wasserstoff, C₁-C₈-Alkyl und/oder C₂-C₈-Alkenyl, wobei diese Reste partiell oder vollständig halogeniert sein und/oder eine bis drei der folgenden Gruppen tragen können: Cyano, C₁-C₄-Alkoxyalkyl, C₁-C₄-Halogenalkyl, C₁-C₄-Alkoxy, C₁-C₄-Halogenalkoxy, C₁-C₄-Alkylthio und C₁-C₄-Alkoxycarbonyl, Aryl und Aryloxy;
Y unabhängig voneinander und von diesen einen der Reste X;
p, q unabhängig voneinander 0, 1, 2;
R⁶ Halogen, Cyano, Nitro, C₁-C₈-Alkyl, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Halogenalkylthio oder eine über Sauerstoff oder Schwefel gebundene Phenylgruppe, welche unsubstituiert ist oder einen bis drei der folgenden Substituenten tragen kann: Halogen, C₁-C₄-Alkyl und C₁-C₄-Alkoxy, wobei R⁶ für r > 1 für verschiedene der genannten Reste stehen kann;
r 0, 1, 2, 3.

2. Verfahren zur Herstellung von Verbindungen der allgemeinen Formel I gemäß Anspruch 1, dadurch gekennzeichnet, daß man eine Carbamoylcarbonsäure der allgemeinen Formel II 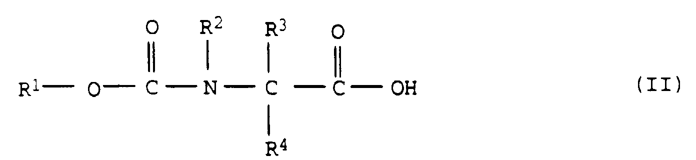 mit einem Amin der allgemeinen Formel III 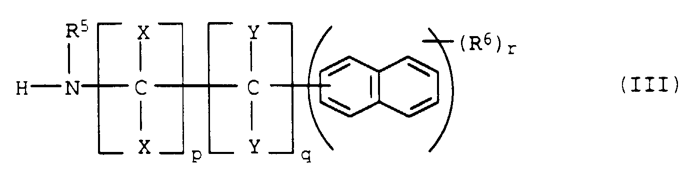 umsetzt.

3. Verfahren zur Herstellung von Verbindungen der allgemeinen Formel I gemäß Anspruch 1, dadurch gekennzeichnet, daß man
a) ein Carbamoyl carbonsäureamid der allgemeinen Formel I 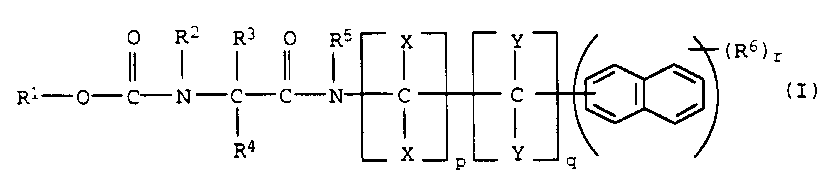 in der die Gruppe R¹-O-(CO) für eine Schutzgruppe steht, die in an sich bekannter Weise abgespalten werden kann, in ein Aminosäureamid IV 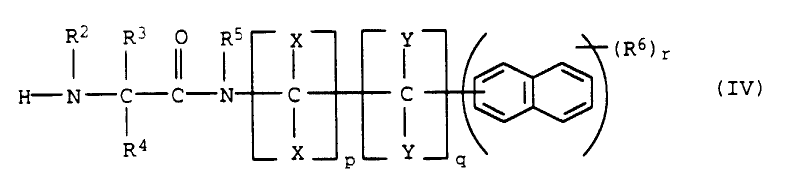 überführt und
b) das so erhaltene Aminosäureamid IV mit einem Chlorameisensäureester der allgemeinen Formel V 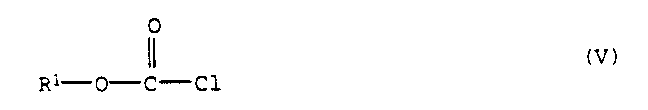 in Gegenwart einer Base umsetzt.

4. Zur Bekämpfung von Schadpilzen geeignete Mittel, enthaltend mindestens einen flüssigen oder festen Trägerstoff und eine fungizid wirksame Menge mindestens einer Verbindung der allgemeinen Formel I oder eines Salzes von I gemäß Anspruch 1.

5. Verfahren zur Herstellung von zur Bekämpfung von Schadpilzen geeigneten Mitteln, dadurch gekennzeichnet, daß man eine fungizid wirksame Menge mindestens einer Verbindung der allgemeinen Formel I oder eines Salzes von I gemäß Anspruch 1 und mindestens einen inerten flüssigen oder festen Trägerstoff sowie gewünschtenfalls mindestens ein Adjuvans mischt.

6. Verfahren zur Bekämpfung von Schadpilzen, dadurch gekennzeichnet, daß man die Schadpilze, deren Lebensraum oder die von ihnen freizuhaltenden Pflanzen, Flächen, Materialien oder Räume mit einer fungizid wirksamen Menge mindestens einer Verbindung der allgemeinen Formel I oder einem ihrer Salze gemäß Anspruch 1 behandelt.

7. Verfahren zur Bekämpfung von Schadpilzen, dadurch gekennzeichnet, daß man die Schadpilze, deren Lebensraum oder die von ihnen freizuhaltenden Pflanzen, Flächen, Materialien oder Räume mit einer fungizid wirksamen Menge mindestens eines Mittels gemäß Anspruch 4 behandelt.

8. Verwendung der Verbindungen der allgemeinen Formel I oder ihren Salzen gemäß Anspruch 1 zur Bekämpfung von Schadpilzen.

9. Verwendung der Mittel gemäß Anspruch 4 zur Bekämpfung von Schadpilzen.

## Claims

1. A carbamoylcarboxamide of the general formula I 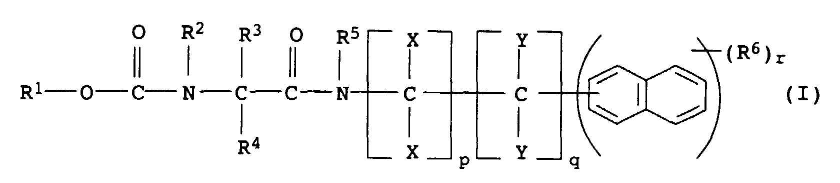 and its salts, where the variables have the following meanings:
R¹ is C₁-C₈-alkyl, C₂-C₈-alkenyl or C₂-C₈-alkynyl, it being possible for these radicals to be partially or completely halogenated and/or to carry one to three of the following groups: cyano, C₁-C₄-alkoxy, C₁-C₄-haloalkoxy, C₁-C₄-alkylthio, C₁-C₄-alkoxycarbonyl, C₃-C₇-cycloalkyl, C₃-C₇-cycloalkenyl, aryl, aryloxy and heteroaryl, it being possible for the cyclic and aromatic rings of these groups in turn to carry one to three of the following substituents: halogen, cyano, C₁-C₄-alkyl, C₁-C₄-alkoxyalkyl, C₁-C₄-haloalkyl, C₁-C₄-alkoxy, C₁-C₄-haloalkoxy, C₁-C₄-alkylthio, C₁-C₄-alkoxycarbonyl, aryl, aryloxy and heteroaryl,
C₃-C₇-cycloalkyl or C₃-C₇-cycloalkenyl, it being possible for these radicals to be partially or completely halogenated and/or to carry one to three of the following groups: cyano, C₁-C₄-alkyl, C₁-C₄-alkoxyalkyl, C₁-C₄-haloalkyl, C₁-C₄-alkoxy, C₁-C₄-haloalkoxy, C₁-C₄-alkylthio, C₁-C₄-alkoxycarbonyl, aryl, aryloxy and aryl-(C₁-C₄)-alkyl, it being possible for the aromatic rings of these groups in turn to carry one to three of the following substituents: halogen, cyano, C₁-C₄-alkyl, C₁-C₄-alkoxyalkyl, C₁-C₄-haloalkyl, C₁-C₄-alkoxy, C₁-C₄-haloalkoxy, C₁-C₄-alkylthio, C₁-C₄-alkoxycarbonyl, aryl and aryloxy,
a nonaromatic 4- to 8-membered ring which, as ring members, in addition to carbon can further contain one or two of the heteroatoms oxygen, sulfur and nitrogen, it being possible for the carbon atoms in the ring to carry one or two of the following groups: halogen, cyano, C₁-C₄-alkyl, C₁-C₄-alkoxyalkyl, C₁-C₄-haloalkyl, C₁-C₄-alkoxy, C₁-C₄-haloalkoxy, C₁-C₄-alkylthio, C₁-C₄-alkoxycarbonyl, aryl and aryloxy, and the second and any further nitrogen atom as a heteroatom in the ring carrying hydrogen or a C₁-C₄-alkyl group;
R² is hydrogen, or C₁-C₈-alkyl or C₃-C₇-cycloalkyl which can be partially or completely halogenated;
R³ is C₁-C₈-alkyl, it being possible for this radical to carry one to three of the following groups: halogen, cyano, C₁-C₄-alkoxy, C₁-C₄-haloalkoxy, C₁-C₄-alkylthio and C₁-C₄-alkoxycarbonyl,
C₃-C₇-cycloalkyl or phenyl-(C₁-C₄)-alkyl, it being possible for the rings of these radicals to carry one to three of the following groups: halogen, cyano, C₁-C₄-alkyl, C₁-C₄-alkoxyalkyl, C₁-C₄-haloalkyl, C₁-C₄-alkoxy, C₁-C₄-haloalkoxy, C₁-C₄-alkylthio, C₁-C₄-alkoxycarbonyl, aryl and aryloxy;
R⁴ is hydrogen or one of the radicals R³ or
R³ and R⁴, together with the C atom to which they are bonded, are a 4- to 8-membered ring which, as ring members, in addition to carbon can further contain one or two of the heteroatoms oxygen, sulfur and nitrogen, it being possible for the carbon atoms in the ring to carry one or two of the following groups: halogen, cyano, C₁-C₄-alkyl, C₁-C₄-alkoxyalkyl, C₁-C₄-haloalkyl, C₁-C₄-alkoxy, C₁-C₄-haloalkoxy, C₁-C₄-alkylthio, C₁-C₄-alkoxycarbonyl, aryl and aryloxy, and nitrogen as a heteroatom carrying hydrogen or a C₁-C₄-alkyl group;
R⁵ independently of these is one of the radicals R²;
X independently of one another is hydrogen, C₁-C₈-alkyl and/or C₂-C₈-alkenyl, it being possible for these radicals to be partially or completely halogenated and/or to carry one to three of the following groups: cyano, C₁-C₄-alkoxyalkyl, C₁-C₄-haloalkyl, C₁-C₄-alkoxy, C₁-C₄-haloalkoxy, C₁-C₄-alkylthio and C₁-C₄-alkoxycarbonyl, aryl and aryloxy;
Y independently of one another and of these is one of the radicals X;
p, q independently of one another are 0, 1 or 2;
R⁶ is halogen, cyano, nitro, C₁-C₈-alkyl, C₁-C₄-alkoxyalkyl, C₁-C₄-haloalkyl, C₁-C₄-alkoxy, C₁-C₄-haloalkoxy, C₁-C₄-alkylthio, C₁-C₄-haloalkylthio or a phenyl group bonded via oxygen or sulfur, which is unsubstituted or can carry one to three of the following substituents: halogen, C₁-C₄-alkyl or C₁-C₄-alkoxy, where R⁶ for r > 1 can be various radicals from those mentioned;
r is 0, 1, 2 or 3.

2. A process for preparing compounds of the general formula I as claimed in claim 1, which comprises reacting a carbamoylcarboxylic acid of the general formula II 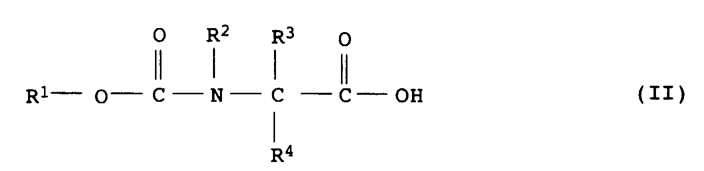 with an amine of the general formula III

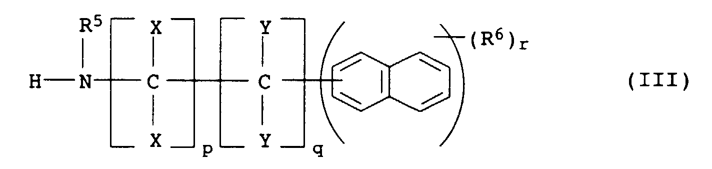

3. A process for preparing compounds of the general formula I as claimed in claim 1, which comprises
a) converting a carbamoylcarboxamide of the general formula I 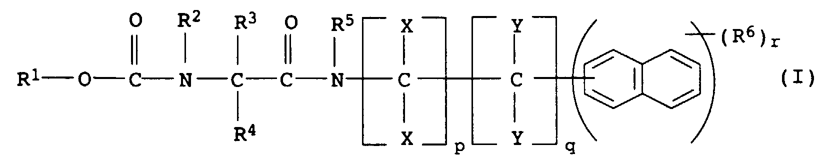 where the group R¹-O-(CO) is a protective group which can be removed in a manner known per se, to an amino acid amide IV 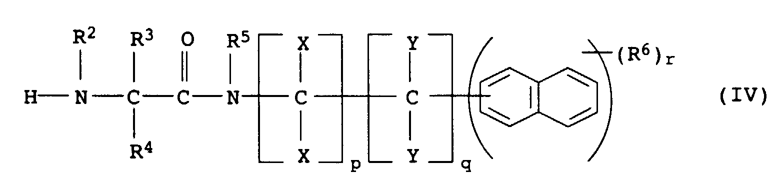 and
b) reacting the amino acid amide IV thus obtained with a chloroformic acid ester of the general formula V 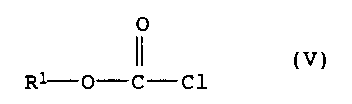 in the presence of a base.

4. A composition suitable for controlling harmful fungi, containing at least one liquid or solid carrier and a fungicidally effective amount of at least one compound of the general formula I or a salt of I as claimed in claim 1.

5. A process for preparing compositions suitable for controlling harmful fungi, which comprises mixing a fungicidally effective amount of at least one compound of the general formula I or a salt of I as claimed in claim 1 and at least one inert liquid or solid carrier and, if desired, at least one adjuvant.

6. A method of controlling harmful fungi, which comprises treating the harmful fungi, their habitat or the plants, surfaces, materials or spaces to be kept free from them with a fungicidally effective amount of at least one compound of the general formula I or one of its salts as claimed in claim 1.

7. A method of controlling harmful fungi, which comprises treating the harmful fungi, their habitat or the plants, surfaces, materials or spaces to be kept free from them with a fungicidally effective amount of at least one composition as claimed in claim 4.

8. The use of the compounds of the general formula I or their salts as claimed in claim 1 for controlling harmful fungi.

9. The use of the compositions as claimed in claim 4 for controlling harmful fungi.

## Revendications

1. Amides d'acides carbamoylcarboxyliques de formule générale I 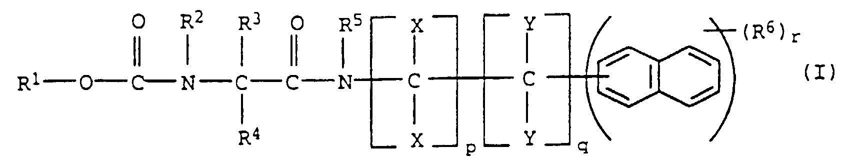 et leurs sels, les symboles de la formule ayant les significations suivantes :
R¹ un groupe alkyle en C1-C8, alcényle en C2-C8 ou alcynyle en C2-C8, ces groupes pouvant être halogénés en totalité ou en partie et /ou porter un à trois des substituants suivants : cyano, alcoxy en C1-C4, halogénoalcoxy en C1-C4, alkylthio en C1-C4, (alcoxy en C1-C4)carbonyle, cycloalkyle en C3-C7, cycloalcényle en C3-C7, aryle, aryloxy et hétéro-aryle, les noyaux alicycliques et aromatiques de ces groupes pouvant eux-mêmes porter un à trois des substituants suivants : halogéno, cyano, alkyle en C1-C4, (alcoxy en C1-C4)-alkyle, halogénoalkyle en C1-C4, alcoxy en C1-C4, halogénoalcoxy en C1-C4, alkylthio en C1-C4, (alcoxy en C1-C4)carbonyle, aryle, aryloxy et hétéroaryle,
un groupe cycloalkyle en C3-C7 ou cycloalcényle en C3-C7 qui peut être partiellement ou totalement halogéné et/ou porter un à trois des substituants suivants : cyano, alkyle en C1-C4, (alcoxy en C1-C4)alkyle, halogénoalkyle en C1-C4, alcoxy en C1-C4, halogéno-alcoxy en C1-C4, alkylthio en C1-C4, (alcoxy en C1-C4)carbonyle, aryle, aryloxy et arylalkyle en C1-C4, les noyaux aromatiques de ces groupes pouvant eux-mêmes porter un à trois des substituants suivants : halogéno, cyano, alkyle en C1-C4, (alcoxy en C1-C4)-alkyle, halogénoalkyle en C1-C4, alcoxy en C1-C4, halogénoalcoxy en C1-C4, alkylthio en C1-C4, (alcoxy en C1-C4)carbonyle, aryle et aryloxy,
un cycle non aromatique de 4 à 8 chaînons qui peut contenir, en tant que chaînons cycliques, avec le carbone, encore un ou deux des hétéroatomes oxygène, soufre et azote, les atomes de carbone cycliques pouvant porter un ou deux des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle et aryloxy, sous réserve que le deuxième atome d'azote et tout autre atome d'azote constituant un hétéroatome cyclique, porte l'hydrogène ou un groupe alkyle en C1-C4 ;
R² représente l'hydrogène, un groupe alkyle en C1-C8 ou cycloalkyle en C3-C7 qui peut être partiellement ou totalement halogéné :
R³ représente un groupe alkyle en C1-C8 qui peut lui-même porter un à trois des substituants suivants : halogéno, cyano, alcoxy en C1-C4, halogénoalcoxy en C1-C4, alkylthio en C1-C4 et (alcoxy en C1-C4)carbonyle,
un groupe cycloalkyle en C3-C7 ou phényl-alkyle en C1-C4, les cycles de ces radicaux pouvant porter un à trois des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle et aryloxy ;
R⁴ : l'hydrogène ou l'une des significations de R³, ou bien
R³ et R⁴, avec l'atome de carbone auquel ils sont reliés, forment un cycle de quatre à huit chaînons qui, en plus du carbone, peut contenir, en tant que chaînons cycliques, un ou deux des hétéroatomes oxygène, soufre et azote, les atomes de carbone cycliques pouvant porter un ou deux des substituants suivants : halogéno, cyano, alkyle en C1-C4, (alcoxy en C1-C4)alkyle, halogénoalkyle en C1-C4, alcoxy en C1-C4, halogénoalcoxy en C1-C4, alkylthio en C1-C4, (alcoxy en C1-C4)carbonyle, aryle et aryloxy, l'azote présent en tant qu'hétéroatome portant l'hydrogène ou un groupe alkyle en C1-C4 ;
R⁵, indépendamment de R², a l'une des significations de ce dernier;
les symboles X représentent chacun, indépendamment l'un de l'autre, l'hydrogène, un groupe alkyle en C1-C8 et/ou alcényle en C2-C8, ces groupes pouvant être partiellement ou totalement halogénés et/ou porter un à trois des substituants suivants : cyano, (alcoxy en C1-C4)alkyle, halogénoalkyle en C1-C4, alcoxy en C1-C4, halogénoalcoxy en C1-C4, alkylthio en C1-C4 et (alcoxy en C1-C4)carbonyle, aryle et aryloxy ;
les symboles Y, indépendamment l'un de l'autre, et indépendamment de X, on chacun l'une des significations de X ;
p et q sont égaux chacun, indépendamment l'un de l'autre, à 0, 1, 2 ;
R⁶ représente un halogène, un groupe cyano, nitro, alkyle en C1-C8, (alcoxy en C1-C4)-alkyle, halogénoalkyle en C1-C4, alcoxy en C1-C4, halogénoalcoxy en C1-C4, alkylthio en C1-C4, halogénoalkylthio en C1-C4 ou un groupe phényle relié par l'intermédiaire de l'oxygène ou du soufre, qui est non substitué ou peut porter un à trois des substituants suivants : halogéno, alkyle en C1-C4 et alcoxy en C1-C4, les symboles R⁶, lorsque r > 1, pouvant avoir des significations différentes ;
r est égal à 0, 1, 2, 3.

2. Procédé de préparation des composés de formule générale I selon la revendication 1, caractérisé par le fait que l'on fait réagir un acide carbamoylcarboxylique de formule générale II 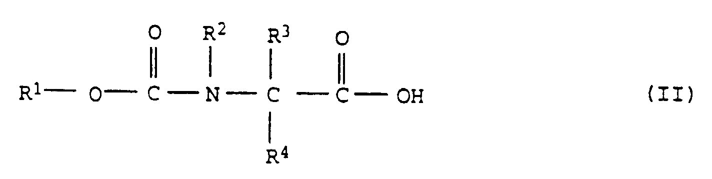 avec une amine de formule générale III

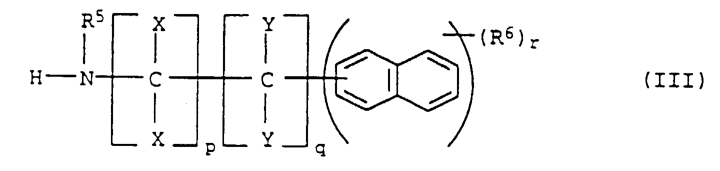

3. Procédé de préparation des composés de formule générale I selon la revendication 1, caractérisé par le fait que
a) on convertit un amide d'acide carbamoylcarboxylique de formule générale I 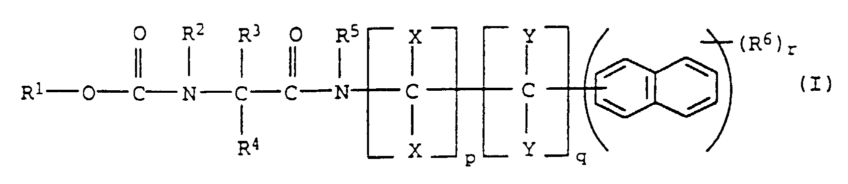 dans laquelle le groupe R¹-O-(CO) est un groupe protecteur qui peut être scindé de manière connue en soi, en un amide d'aminoacide IV 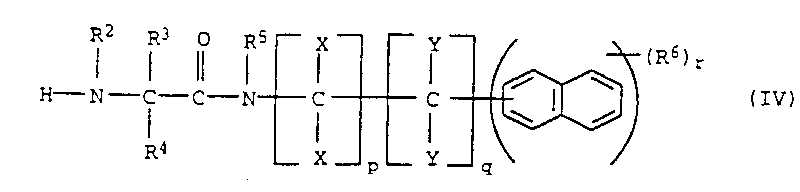 et,
b) on fait réagir cet amide d'aminoacide IV avec un ester chloroformique de formule générale V 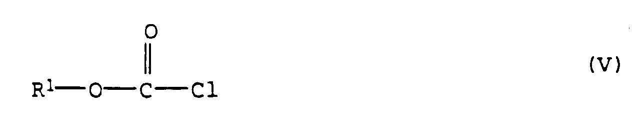 en présence d'une base.

4. Produits appropriés à l'utilisation pour la lutte contre les mycètes nuisibles, contenant au moins un véhicule liquide ou solide et une quantité fongicide efficace d'au moins un composé de formule générale I ou d'un sel de I selon la revendication 1.

5. Procédé pour la préparation de produits appropriés à l'utilisation pour la lutte contre les mycètes nuisibles, caractérisé par le fait que l'on mélange une quantité fongicide efficace d'au moins un composé de formule générale I ou d'un sel de I selon la revendication 1 et au moins un véhicule liquide ou solide inerte et, si on le désire, au moins un adjuvant.

6. Procédé pour combattre les mycètes nuisibles, caractérisé par le fait que l'on traite ces mycètes, leur habitat ou les végétaux, aires, matériaux ou locaux contre lesquels on veut les protéger par une quantité fongicide efficace d'au moins un composé de formule générale I ou de l'un de ses sels selon la revendication 1.

7. Procédé pour combattre les mycètes nuisibles, caractérisé par le fait que l'on traite les mycètes nuisibles, leur habitat ou les végétaux, aires, matériaux ou locaux contre lesquels on veut les protéger par une quantité fongicide efficace d'au moins un produit selon la revendication 4.

8. Utilisation des composés de formule générale I ou de leurs sels selon la revendication 1 pour la lutte contre les mycètes nuisibles.

9. Utilisation des produits selon la revendication 4 pour la lutte contre les mycètes nuisibles.
